# EUROPEAN PATENT SPECIFICATION

(11) **EP 1 089 757 B1**
(45) Date of publication and mention of the grant of the patent: **22.04.2009**
(21) Application number: 99928792.3
(22) Date of filing: 17.06.1999
(51) Int. Cl.: A61K 39/145, C07K 7/06

(54) **HLA BINDING PEPTIDES AND THEIR USES**
HLA-BINDENDE PEPTIDE UND IHRE VERWENDUNGEN
PEPTIDES LIANT HLA-A2 ET LEURS UTILISATIONS

(30) Priority: 17.06.1998 US 98584
(43) Date of publication of application: 11.04.2001
(60) Divisional application: 08018213.2
(73) Proprietor: IDM Pharma, Inc., Irvine, CA 92618-1605 (US)
(72) Inventor: SETTE, Alessandro, La Jolla, CA 92037 (US); SIDNEY, John, San Diego, CA 92130 (US); SOUTHWOOD, Scott, Santee, CA 92071 (US)
(74) Representative: Cripps, Joanna Elizabeth
(86) International application number: PCT/US1999/013789
(87) International publication number: WO 1999/065522

(56) References cited:
- WO-A-94/03205
- WO-A-94/20127
- WO-A-96/18409
- WO-A-97/29195
- WO-A-97/41440
- WO-A2-93/20103
- US-A- 5 686 068
- RAMMENSEE H-G ET AL: "MHC LIGANDS AND PEPTIDE MOTIFS: FIRST LISTING" IMMUNOGENETICS, SPRINGER VERLAG, BERLIN, DE, vol. 41, no. 4, 1 February 1995 (1995-02-01), pages 178-228, XP000673045 ISSN: 0093-7711
- KAWASHIMA I ET AL: "THE MULTI-EPITOPE APPROACH FOR IMMUNOTHERAPY FOR CANCER: IDENTIFICATION OF SEVERAL CTL EPITOPES FROM VARIOUS TUMOR-ASSOCIATED ANTIGENS EXPRESSED ON SOLID EPITHELIAL TUMORS" HUMAN IMMUNOLOGY, NEW YORK, NY, US, vol. 59, no. 1, January 1998 (1998-01), pages 1-14, XP001012509 ISSN: 0198-8859
- RAS E ET AL: "IDENTIFICATION OF POTENTIAL HLA-A *0201 RESTRICTED CTL EPITOPES DERIVED FROM THE EPITHELIAL CELL ADHESION MOLECULE (EP-CAM) AND THECARCINOEMBRYONIC ANTIGEN (CEA)" HUMAN IMMUNOLOGY, NEW YORK, NY, US, vol. 53, 1997, pages 81-89, XP002923757 ISSN: 0198-8859
- ZAREMBA S ET AL: "Identification of an enhancer agonist cytotoxic T lymphocyte peptide from human carcinoembryonic antigen" CANCER RESEARCH, AMERICAN ASSOCIATION FOR CANCER RESEARCH, BALTIMORE, MD, US, vol. 57, 15 October 1997 (1997-10-15), pages 4570-4577, XP002096107 ISSN: 0008-5472
- NIJMAN H W ET AL: "CHARACTERIZATION OF CYTOTOXIC T LYMPHOCYTE EPITOPES OF A SELF-PROTEIN, P53, AND A NON-SELF-PROTEIN, INFLUENZA MATRIX: RELATIONSHIP BETWEEN MAJOR HISTOCOMPATIBILITY COMPLEX PEPTIDE BINDING AFFINITY AND IMMUNE RESPONSIVENESS TO PEPTIDES" JOURNAL OF IMMUNOTHERAPY, RAVEN PRESS, NEW YORK, US, vol. 14, no. 2, 1993, pages 121-126, XP002015226 ISSN: 1053-8550
- RUPPERT J ET AL: "PROMINENT ROLE OF SECONDARY ANCHOR RESIDUES IN PEPTIDE BINDING TO HLA-A2.1 MOLECULES" CELL, CELL PRESS, CAMBRIDGE, NA, US, vol. 74, no. 5, 10 September 1993 (1993-09-10), pages 929-937, XP000609144 ISSN: 0092-8674
- PARKER K C ET AL: "SCHEME FOR RANKING POTENTIAL HLA-A2 BINDING PEPTIDES BASED ON INDEPENDENT BINDING OF INDIVIDUAL PEPTIDE SIDE-CHAINS" JOURNAL OF IMMUNOLOGY, WILLIAMS & WILKINS CO, US, vol. 152, no. 1, January 1994 (1994-01), pages 163-175, XP000884375
- WYSOCKA M ET AL: "Class I H-2d-restricted cytotoxic T lymphocytes recognize the neuraminidase glycoprotein of influenza virus subtype N1." JOURNAL OF VIROLOGY, (1990 MAR) 64 (3) 1028-32., XP001057676
- RAMMENSEE HANS-GEORG ET AL: "SYFPEITHI: Database for MHC ligands and peptide motifs" IMMUNOGENETICS, SPRINGER VERLAG, BERLIN, DE, vol. 50, no. 3-4, November 1999 (1999-11), pages 213 &-219, XP002183663 ISSN: 0093-7711
- BLOK J. ET AL: 'Sequence Variation at the 3' End of the Neuraminidase Gene from 39 Influenza Type A Viruses' VIROLOGY vol. 121, 1982, pages 211 - 229, XP002924632
- HOUBIERS J.G.A. ET AL: 'In vitro induction of human cytotoxic T lymphocyte responses against peptides of mutant and wild-type p53' EUROPEAN JOURNAL OF IMMUNOLOGY vol. 23, 1993, pages 2072 - 2077
- THEOBALD M. ET AL: 'Tageting p53 as a general tumor antigen' PNAS vol. 92, December 1995, pages 11993 - 11997

## Description

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either class I or class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptideMHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and α2 domains of the class I heavy chain (Bjorkman et al., Nature 329:506 (1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC class I has been presented by Rötzschke and Falk (Rötzschke & Falk, Immunol. Today 12:447 (1991).

Sette et al., Proc. Natl. Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J. Immunol., 21:2963-2970 (1991); Pamer et al., Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

Despite the developments in the art, the prior art has yet to provide a useful human peptide-based vaccine or therapeutic agent based on this work. The present invention provides these and other advantages.

### SUMMARY OF THE INVENTION

The present invention provides an isolated immunogenic peptide consisting of p53 sequence SMPPPGTRV (SEQ ID NO:4). Also described herein are immunogenic peptides having allele specific binding motifs, such binding motifs for HLA-A2.1 molecules. The immunogenic peptides, which hind to the appropriate MHC allele, are preferably 9 to 10 residues in length and comprise conserved residues at certain positions such as positions 2 and 9. Moreover, the peptides do not comprise negative binding residues as defined herein at other positions such as positions 1, 3, 6 and/or 7 in the case of peptides 9 amino acids in length and positions 1, 3, 4, 5, 7, 8 and/or 9 in the case of peptides 10 amino acids in length. The present invention defines positions within a motif enabling the selection of peptides which will bind efficiently to HLA A2.1.

The motifs of the peptides described herein include peptides of 9 amino acids which have a first conserved residue at the second position from the N-terminus selected from the group consisting of L, M, I, V, A, T, and Q and a second conserved residue at the C-terminal position selected from the group consisting of V, L, I, A, M, and T. In a preferred embodiment, the peptide may have a first conserved residue at the second position from the N-terminus selected from the group consisting of V, A, T, or Q; and a second conserved residue at the C-terminal position selected from the group consisting of L, M, I, V, A, and T. If the peptide has 10 residues it will contain a first conserved residue at the second position from the N-terminus selected from the group consisting of L, M, I, V, A, T, and Q; and a second conserved residue at the C-terminal position selected from the group consisting of V, I. L, A, T, and M; wherein the first and second conserved residues are separated by 7 residues.

The present invention also provides compositions comprising the immunogenic peptide consisting of p53 sequence SMPPPGTRV (SEQ ID NO:4). The composition may further comprise a further immunogenic peptide. The immunogenic peptides are typically between about 8 and about 11 residues and comprise conserved residues involved in binding proteins encoded by the appropriate MHC allele. A number of allele specific motifs have been identified.

For instance, the motif for HLA-A3.2 comprises from the N-terminus to C-terminus a first conserved residue of L, M, I, V, S, A, T, F, C, G, or D at position 2 and a second conserved residue of K, R, Y, H, F, or A at the C-terminal end. The first and second conserved residues are preferably separated by 6 to 7 residues.

The motif for HLA-A1 comprises from the N-teminus to the C-terminus a first conserved residue at position two relative to the N-terminus ofT, S or M, and a second conserved residue of Y at the C-terminus. Alternatively, the peptide may have a conserved residue of D, E, A, S, or T at position 3 relative to the N-terminus and a conserved residue of Y at the C-terminus.

The motif for HLA-A11 comprises from the N-terminus to the C-terminus a first conserved residue of T, V, M, L, I, S, A, G, N, C D, or F at position 2 and a C-terminal conserved residue of K, R, Y or H. The first and second conserved residues are preferably separated by 6 or 7 residues.

The motif for HLA-A24.1 comprises from the N-terminus to the C-terminus a first conserved residue of Y, F, W, or M at position 2 and a C terminal conserved residue of F, I, W, or L. The first and second conserved residues are preferably separated by 6 to 7 residues.

Epitopes on a number of immunogenic target proteins can be identified using the peptides of the invention. Examples of suitable antigens include prostate cancer specific antigen (PSA), hepatitis B core and surface antigens (HBVc, HBVs) hepatitis C antigens, Epstein-Barr virus antigens, human immunodeficiency type-1 virus (HIV1), Kaposi's sarcoma herpes virus (KSHV), human papilloma virus (HPV) antigens, Lassa virus, mycobacterium tuberculosis (MT), p53, CEA, trypanosome surface antigen (TSA) and Her2/neu. The peptides are thus useful in pharmaceutical compositions for both *in vivo* and ex vivo therapeutic and diagnostic applications.

### DRAWINGS

Not applicable.

### DEFINITIONS

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind an MHC molecule and induce a CTL response. Immunogenic peptides of the invention are capable of binding to an appropriate HLA-A2.1 molecule and inducing a cytotoxic T cell response against the antigen from which the immunogenic peptide is derived.

Immunogenic peptides are conveniently identified using the algorithms described herein. The algorithms are mathematical procedures that produce a score which enables the selection of immunogenic peptides. Typically one uses the algorithmic score with a "binding threshold" to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

A "conserved residue" is an amino acid which occurs in a significantly higher frequency than would be expected by random distribution at a particular position in a peptide. Typically a conserved residue is one where the MHC structure may provide a contact point with the immunogenic peptide. At least one to three or more, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein. "negative binding residues" are amino acids which if present at certain positions (for example, positions 1, 3 and/or 7 of a 9-mer) will result in a peptide being a nonbinder or poor binder and in turn fail to be immunogenic i-e. induce a CTL response.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues and negative residues.

The binding motif for an allele can be defined with increasing degrees of precision. In one case, all of the conserved residues are present in the correct positions in a peptide and there are no negative residues in positions 1,3 and/or 7.

The phrases "isolated" or "biologically pure" refer to material which is substantially or essentially free from components which normally accompany it as found in its native slate. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### DESCRIPTION OF THE PREFERRED EMBODIMENTS

The present invention relates to the determination of allele-specific peptide motifs for human class I MHC (sometimes referred to as HLA) allele subtypes. These motifs are then used to define T cell epitopes from any desired antigen, particularly those associated with human viral diseases, cancers or autoiummune diseases, for which the amino acid sequence of the potential antigen or autoantigen targets is known.

Epitopes on a number of potential target proteins can be identified in this manner. Examples of suitable antigens include prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens, human papilloma virus (HPV) antigens, Lassa virus, mycobacterium tuberculosis (MT), p53, CEA, trypanosome surface antigen (TSA) and Her2/neu.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary *in vitro* or *in vivo* CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fold lower). Each of these loci have a number of alleles. The peptide binding motifs of the invention are relatively specific for each allelic subtype.

For peptide-based vaccines, the peptides preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypt:s, specifically HLA-A2.1, A1, A3.2, and A24.1. Similarly, the majority of the Asian population is encompassed with the addition of peptides binding to a fifth allele HLA-A 11.2.

**TABLE 1**

| A Allele/Subtype | N(69)* | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

Table compiled from DuPont, Immunobiology of HLA, Vol. 1, Histocompatibility Testing 1987, Springer-Verlag, New York 1989.
* N - negroid; A = Asian; C = caucasoid. Numbers in
   Parenthesis represent the number of individuals included in the analysis.

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The procedures used to identify peptides of the present invention generally follow the methods disclosed in Falk et al., Nature 351:290 (1991), Briefly, the methods involve large-scale isolation of MHC class I molecules, typically by immunoprecipitation or affinity chromatography, from the appropriate cell or cell line. Examples of other methods for isolation of the desired MHC molecule equally well known to the artisan include ion exchange chromatography, lectin chromatography, size exclusion, high performance ligand chromatography, and a combination of all of the above techniques.

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B1, and HLA-C molecules. Several mAb reagents for the isolation of HLA-A molecules are available (*see* Table 2). Thus, for each of the targeted HLA-A alleles, reagents are available that may be used for the direct isolation of the HLA-A molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and B9.12.1, and one anti-HLA-B, C mAb, B 1.23.2, could be used in alternative affinity purification protocols as described in previous applications.

**TABLE 2**

| ANTIBODY REAGENTS | | |
|---|---|---|
| anti-HLA | Name | |
| HLA-A1 | 12/ 18 | |
| HLA-A2 | BB7.2 | |
| HLA-A3 | GAPA3 | (ATCC, HB 122) |
| HLA-11.24.1 | A11.1 M | (ATCC, HB164) |
| HLA-A, B, C | W6/32 | (ATCC, HB95) |
| HLA-A, B, C | B9.12.1 | (INSERM-CNRS)_{.} |
| HLA-B, C | B.1.23.2 | (INSERM-CNRS) |

The peptides bound to the peptide binding groove of the isolated MHC molecules are eluted typically using acid treatment. Peptides can also be dissociated from class molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration) electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, et al., Methods Enzymol. 91, 399 (1983)). Other methods suitable for sequencing include mass spectrometry sequencing of individual peptides as previously described (Hunt, et al., Science 225:1261 (1992), ). Amino acid sequencing of bulk heterogenous peptides (e.g., pooled HPLC fractions) from different class I molecules typically reveals a characteristic sequence motif for each class I allele.

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecule binding assay as described in the related applications, noted above. Other alternatives described in the literature include inhibition of antigen presentation (Sette, et al., J. Immunol. 141:3893 (1991), *in vitro* assembly assays (Townsend, et al.. Cell 62:285 (1990), and FACS based assays using mutated ells, such as RMA-S (Melief, et al., Eur. J. Immunol. 21:2963 (1991)).

Next, peptides that test positive in the MHC class I binding assay are assayed for the ability of the peptides to induce specific CTL responses *in vitro.* For instance, Antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 (1988)).

Alternatively, mutant mammalian cell lines that are deficien in their ability to load class I molecules with internally processed peptides, such as the mouse cell lines RMA-S (Kärre, et al.. Nature, 319:675 (1986); Ljunggren, et al., Eur. J. Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce *in vitro* primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (*see* Schneider, J. Embryol. Exp. Morphol. 27:353-365 (1927)).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations *in vitro* for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of 9 or 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany & Merrifield, The Peptides, Gross & Meienhofer, eds. (N.Y., Academic Press), pp. 1-284 (1979); and Stewart & Young, Solid Phase Peptide Synthesis, (Rockford, III., Pierce), 2d Ed. (1984).

The peptides can also be modified by extending or decreasing the compound's amino acid sequence, e.g., by the addition or deletion of amino acids. The peptides or analogs can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-y-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 3 when it is desired to finely modulate the characteristics of the peptide.

**TABLE 3**

| Original Residue | Exemplary Substitution |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |
| Pro | Gly |

Substantial changes in function (e.g., affinity for MHC molecules or T cell receptors) are made by selecting substitutions that are less conservative than those in Table 3, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, Peptides and Proteins, Vol. VII (Weinstein ed., 1983).

Modifications of peptides with various amino acid mimetics or unnatural amino acids are particularly useful in increasing the stability of the peptide *in vivo.* Stability can be assayed in a number of ways. For instance, peptidases and various biological media, such as human plasma and serum, have been used to test stability. *See, e.g.,* Verhoef et al., Eur. J. Drug Metab Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

A large number of cells with defined MHC molecules, particularly MHC Class I molecules, are known and readily available. For example, human EBV-transformed B cell lines have been shown to be excellent sources for the preparative isolation of class I and class II MHC molecules. Well-characterized cell lines are available from private and commercial sources, such as American Type Culture Collection ("Catalogue of Cell Lines and Hybridomas," 6th edition (1988) Rockville, Maryland, U.S.A.); National Institute of General Medical Sciences 1990/1991 Catalog of Cell Lines (NIGMS) Human Genetic Mutant Cell Repository, Camden, NJ; and ASHI Repository, Brigham and Women's Hospital, 75 Francis Street, Boston, MA 02115. Table 4 lists some B cell lines suitable for use as sources for HLA-A alleles. All of these cell lines can be grown in large batches and are therefore useful for large scale production of MHC molecules. One of skill will recognize that these are merely exemplary cell lines and that many other cell sources can be employed. Similar EBV B cell lines homozygous for HLA-B and HLA-C could serve as sources for HLA-B and HLA-C alleles, respectively.

**TABLE 4**

| HUMAN CELL LINES (HLA-A SOURCES) | |
|---|---|
| HLA-A allele | B cell line |
| A 1 | MAT |
| | COX (9022) |
| | STEINLIN (9087) |
| A2.1 | JY |
| A3.2 | EHM (9080) |
| | HO301 (9055) |
| | GM3107 |
| A24.1 | KT3(9107),TISI (9042) |
| A11 | BVR(GM6828A) |
| | WT100 (GM8602) |
| | WT52 (GM8603) |

The peptides or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helper peptides include tetanus toxoid 830-843, influenza 307-319, malaria circumsporozoite 382-398 and 378-389

In some embodiments it may be desirable to include in the pharmaceutical compositions of the invention at least one components which assists in priming CTL. Lipids have been identified as agents capable of assisting the priming CTL *in vivo* against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P3CSS) can be used to prime virus specific CTL when covalently attached to an appropriate peptide. See, Deres et al., Nature 342:561-564 (1989),. Peptides can be coupled to P3CSS, for example, and the lipopeptide administered to an individual to specifically prime a CTL response to the target antigen. Further, as the induction of neutralizing antibodies can also be primed with P3CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH2 acylation, e.g., by alkanoyl (C1-C20) or thioglycolyl acetylation, terminal-carboxyl amidation, e.g., ammonia, methylamine, etc. In some instances these modifications may provide sites for linking to a support or other molecule.

The peptides including SMPPPGTRV (SEQ ID NO:14) can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart & Young, Solid Phase Peptide Synthesis, 2d. ed., Pierce Chemical Co. (1984).

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982). Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can.be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptide of the present invention and pharmaceutical and vaccine compositions comprising said peptide are useful for administration to mammals, particularly humans, to treat and/or prevent viral infection and cancer. Examples of diseases which can be treated or prevented using the immunogenic peptides of the invention include prostate cancer, hepatitis B, hepatitis C, HPV infection, AIDS, renal carcinoma, cervical carcinoma, lymphoma, CMV, malaria, and condlyloma acuminatum.

Vaccines that contain an immunenically effective amount of the peptide of the invention and optionally other peptides as described herein are a further embodiment of the invention. Once appropriately immunogenic epitopes have been defined, they can be sorted and delivered by various means, herein referred to as "vaccine" compositions. Such vaccine compositions can include, for example, lipopeptides (Vitiello, A. et al., J. Clin. Invest. 95:341, 1995), peptide compositions encapsulated in poly(DL-lactide-co-glycolide) ("PLG") microspheres (*see, e.g.,* Eldridge, et al., Molec. Immunol. 28:287-294 (1991); Alonso et al., Vaccine 12:299-306, 1994; Jones et al., Vaccine 13:675-681 (1995)), peptide compositions contained in immune stimulating complexes (ISCOMS) (*see, e.g.,* Takahashi et al., Nature 344:873-875 (1990); Hu et al., Clin Exp Immunol. 113:235-243 (1998)), multiple antigen peptide systems (MAPs) *(see e.g.,* Tarn, Proc. Natl. Acad. Sci. U.S.A. 85:5409-5413 (1988); Tam, J. Immunol. Methods 196:17-32 (1996)), viral delivery vectors (Perkus, et al., In: Concepts in vaccine development, Kaufmann, ed., p. 379 (1996); Chakrabarti, et al., Nature 320:535 (1986); Hu, et al., Nature 320:537 (1986); Kieny, et al., AIDS Bio/Technology 4:790 (1986); Top et al. , J. Infect. Dis. 124:148 (1971); Chanda, et al., Virology 175:535 (1990)), particles of viral or synthetic origin (Kofler, et al., J. Immunol. Methods. 192:25 (1996); Eldridge, et al., Sem, Hematol. 30:16 (1993); Falo et al., Nature Med. 7:649 (1995)), adjuvants (Warren, et al., Annu. Rev. Immunol. 4:369 (1986); Gupta et al., Vaccine 11:293 (1993)), liposomes (Reddy et al., J. Immunol. 148:1585 (1992); Rock, Immunol. Today 17;131 (1996)), or, naked or particle absorbed cDNA (Ulmer et al., Science 259:1745 (1993); Robinson i., Vaccine 11:957 (1993); Shiver et al., In: Concepts in vaccine development, Kaufmann, ed., p. 423, (1996); Cease & Berzofsky Annu. Rev. Immunol. 12:923 (1994) and Eldridge et al., Sem, Hematol. 30:16 (1993)). Toxin-targeted delivery technologies, also known as receptor mediated targeting, such as those of Avant Immunotherapeutics. Inc. (Needham, Massachusetts) may also be used.

Furthermore, vaccines in accordance with the invention encompass compositions. comprising the claimed peptide. The peptide can be individually linked to its own carrier; alternatively, the peptide can exist as a homopolymer or heteropolymer of active peptide units. Such a polymer has the advantage of increased immunological reaction and, where different peptide epitopes are used to make up the polymer, the additional ability to induce antibodies and/or CTLs that react with different antigenic determinants from one or multiple proteins of the pathogenic organism or tumor-related peptide targeted for an immune response.

Useful carriers that can be used with vaccines of the invention are well known in the art, and include, *e.g*., thyroglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (i.e.. acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, as disclosed herein, CTL responses can be primed by conjugating peptides of the invention to lipids, such as tripalmitoyl-S-glycerylcysteinlyseryl- serine (P₃CSS).

As disclosed in greater detail herein, upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs specific for the desired antigen. Consequently, the host becomes at least partially immune to later infection, or at least partially resistant to developing an ongoing chronic infection, or derives at least some therapeutic benefit when the antigen was tumor-associated.

In some instances it may be desirable to combine the class I peptide vaccines with vaccines which induce or facilitate neutralizing antibody responses to the target antigen of interest, particularly to viral envelope antigens. A preferred composition comprises class I and class II epitopes in accordance with the invention. An alternative of such a composition comprises a class I and/or class II epitope in accordance with the invention, along with a PADRE™ (Epimmune, San Diego, CA) molecule (described in U.S. Patent Number 5,736,142). Furthermore, any of these can be administered as a nucleic acid mediated modality.

Pharmaceutical compositions comprising the immunogenic peptide of the invention may be administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 50,000 µg of peptide for a 70 kg patient, followed by boosting dosages or from about 1.0 µg to about 10,000 µg of peptide pursuant to a boosting regimen over weeks to months depending upon the patient's response and condition by measuring specific CTL activity in the patient's blood. It must be kept in mind that the peptide and compositions of the present invention may generally be employed in serious disease states, that is, life-threatening or potentially life threatening situations. In such cases, in view of the minimization of extraneous substances and the relative nontoxic nature of the peptide, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 50,000 µg, preferably about 5 µg to 10,000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptide of the invention may also be administered via liposomes, which target the peptides to a particular cells tissue, such as lymphoid tissue. Liposomes are also useful in increasing the half-life of the peptides. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9;467 (1980), U.S. Patent Nos. 4,235,871, 4,501,728, 4,837,028, and 5,019,369 .

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01 %-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included. as desired, as with, e.g., lecithin for intranasal delivery.

Upon immunization with a peptide composition as described herein, via injection, aerosol, oral, transdermal or other route, the immune system of the host responds to the vaccine by producing large amounts of CTLs specific for the desires antigen, and the host becomes at least partially immune to later infection, or resistant to developing chronic infection.

Vaccine compositions containing the peptide of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 50,000 µg per 70 kilogram patient, more commonly from about 10 µg to about 10,000 µg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, nucleic acids encoding the peptide of the invention and optionally one or more of the peptides described herein can also be administered to the patient. A number of methods are conveniently used to deliver the nucleic acids to the patient. For instance, the nucleic acid can be delivered directly, as "naked DNA". This approach is described, for instance, in Wolff et al., Science 247: 1465-1468 (1990) as well as U.S. Patent Nos. 5,580,859 and 5,589,466. The nucleic acids can also be administered using ballistic delivery as described, for instance, in U.S. Patent No. 5,204,253. Particles comprised solely of DNA can be administered. Alternatively, DNA can be adhered to particles, such as gold particles.

The nucleic acids can also be delivered complexed to cationic compounds, such as cationic lipids. Lipid-mediated gene delivery methods are described, for instance, in WO 96/18372; WO 93/24640; Mannino & Gould-Fogerite , BioTechniques 6(7): 682-691 (1988); Rose U.S. Pat No. 5,279,833; WO 91/06309; and Felgner et al., Proc. Natl. Acad. Sci. USA 84: 7413-7414 (1987).

The peptide of the invention can also be expressed by attenuated viral hosts, such as vaccinia or fowlpox. This approach involves the use of vaccinia virus as a vector to express nucleotide sequences that encode the peptide of the invention. Upon introduction into an acutely or chronically infected host or into a noninfected host, the recombinant vaccinia virus expresses the immunogenic peptide, and thereby elicits a host CTL response. Vaccinia vectors and methods useful in immunization protocols are described in, e.g.,U.S. Patent No. 4,722,848,. Another vector is BCG (Bacille Calmette Guerin). BCG vectors are described in Stover et al. (Nature 351:456-460 (1991)) . A wide variety of other vectors useful for therapeutic administration or immunization of the peptides of the invention, e.g., *Salmonella typhi* vectors and the like, will be apparent to those skilled in the art from the description herein.

A preferred means of administering nucleic acids encoding the peptide of the invention uses minigene constructs encoding multiple epitopes,. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte, epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. he ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E. coli* origin of replication; and an E. coli selectable marker (e.g. ampicillin or kanamycin resistance). Numerous promoters can be used for this purpose, e.g., the human cytomegalovirus (hCMV) promoter. See, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA' vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate E. coli strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in E. coli, followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51 Cr release, indicates production of MHC presentation of mini gene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

Antigenic peptides may be used to elicit CTL *ex vivo,* as well. The resulting CTL, can be used to treat chronic infections (viral or bacterial) or tumors in patients that do not respond to other conventional forms of therapy, or will not respond to a peptide vaccine approach of therapy. *Ex vivo* CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell). In order to optimize the *in vitro* conditions for the generation of specific cytotoxic T cells, the culture of stimulator cells is maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that will allow about 200, and preferably 200 or more, human Class I MHC molecules loaded with peptide to be expressed on the surface of each stimulator cell. Preferably, the stimulator cells are incubated with >20µg/ml peptide.

Resting or precursor CD8+ cells are then incubated in culture with the appropriate stimulator cells for a time period sufficient to activate the CD8+ cells. Preferably, the CD8+ cells are activated in an antigen-specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the within-described treatment modality is used. Preferably, however, the lymphocyte:stimulator cell ratio is in the range of about 30:1 to 300:1. The effector/stimulator culture may be maintained for as long a time as is necessary to stimulate a therapeutically useable or effective number of CD8+ cells.

The induction of CTL *in vitro* requires the specific recognition of peptides that are bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses. Peptide loading of empty major histocompatability complex molecules on cells enables the induction of primary cytotoxic T lymphocyte responses.

Since mutant cell lines do not exist for every human MHC allele, it is advantageous to use a technique to remove endogenous MHC-associated peptides from the surface of APC, followed by loading the resulting empty MHC molecules with the immunogenic peptides of interest. The use of non-transformed (non-tumorigenic), noninfected cells, and preferably, autologous cells of patients as APC is desirable for the design of CTL induction protocols directed towards development of ex vivo CTL therapies. This application discloses methods for stripping the endogenous MHC-associated peptides from the surface of APC followed by the loading of desired peptides.

A stable MHC class I molecule is a trimeric complex formed of the following elements: 1) a peptide usually of 8 - 10 residues, 2) a transmembrane heavy polymorphic protein chain which bears the peptide-binding site in its α1 and α2 domains, and 3) a non-covalently associated non-polymorphic light chain, β2microglobuiin. Removing the bound peptides and/or dissociating the β2microglobulin from the complex renders the MHC class I molecules nonfunctional and unstable, resulting in rapid degradation. All MHC class I molecules isolated from PBMCs have endogenous peptides bound to them. Therefore, the first step is to remove all endogenous peptides bound to MHC class I molecules on the APC without causing their degradation before exogenous peptides can be added to them.

Two possible ways to free up MHC class I molecules of bound peptides include lowering the culture temperature from 37°C to 26°C overnight to destablize β2microglobulin and stripping the endogenous peptides from the cell using a mild acid treatment. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells not actively synthesizing MHC molecules (e.g., resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction, since it is important to remove the endogenous peptides while preserving APC viability and an optimal metabolic state which is critical for antigen presentation. Mild acid solutions of pH 3 such as glycine or citrate-phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen-specific CTL. The resulting APC are efficient in inducing peptide-specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibody-tagged molecules may then be extracted from the stimulator-effector cell admixture via appropriate means, e.g., via well-known immunoprecipitation or immunoassay methods.

Effective, cytotoxic amounts of the activated CD8+ cells can vary between *in vitro* and *in vivo* uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Preferably, as discussed above, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells is not achieved, there is no inherent danger known to be associated with the administration of a small number of stimulator cells, whereas administration of mammalian tumor-promoting cells may be extremely hazardous.

Methods of re-introducing cellular components are known in the art and include procedures such as those exemplified in U.S. Patent No. 4,844,893 to Honsik, et al*.* and U.S. Patent No. 4,690,915 to Rosenberg. For example, administration of activated CD8+ cells via intravenous infusion is appropriate.

The immunogenic peptide of this invention may also be used to make monoclonal antibodies. Such antibodies may he useful as potential diagnostic: or therapeutic agents.

The peptide may also find use as diagnostic reagents. For example, a peptide may be used to determine the susceptibility of a particular individual to a treatment regimen which employs the peptide or related peptides, and thus may he helpful in modifying an existing treatment protocol or in determining a prognosis for an affected individual. In addition, the peptides may also be used to predict which individuals will be at substantial risk for developing chronic infection.

### EXAMPLES

The following examples are provided by way of illustration only and not by way of limitation. Those of skill in the art will readily recognize a variety of noncritical parameters that could be changed or modified to yield essentially similar results,

### Example 1

Class I antigen isolation was carried out as described in the related applications, noted above. Naturally processed peptides were then isolated and sequenced as described there. An allele-specific motif and algorithms were determined and quantitative binding assays were carried out.

Using the motifs identified above for HLA-A2.1 allele amino acid sequences from a number of antigens were analyzed for the presence of these motifs. Table 5 provides the results of these searches.

**Table 5**

| Peptide | AA | Sequence | Source | A*0201 |
|---|---|---|---|---|
| 17.0317 | 9 | LQIGNIISI | Flu.24 | 0.0130 |
| 38.0103 | 9 | NLSLSCHAA | CEA.432 | 0.0110 |
| 1233.11 | 9 | YLSGANLNV | CEA.605V9 | 0.0690 |
| 1295.03 | 9 | SMPPPGTRV | p53.149M2 | 0.0290 |
| 1295.04 | 9 | SLPPPGTRV | p53.149L2 | 0.0410 |
| 1317.24 | 9 | KTCPVQLWV | p53.139 | 0.0069 |
| 1323.02 | 9 | KLLPENNVV | p53.24V9 | 0.0130 |
| 1323.04 | 9 | ALNKMFBQV | p53.129B7V9 | 0.0260 |
| 323.06 | 9 | KLBPVQLWV | p53.139L2B3 | 0.1100 |
| 1323.08 | 9 | BLTIHYNYV | p53.229B1L2V9 | 0.0430 |
| 1323.18 | 10 | LLPPQHLIRV | p53.188L2 | 0.0061 |
| 1323.29 | 11 | YMCNSSCMGGM | p53.236 | 0.0075 |
| 1323.31 | 11 | YLCNSSCMGGV | p53.236L2V11 | 0.2300 |
| 1323.34 | 11 | KLYQGSYGFRV | p53.101L2V11 | 0.0620 |
| 1324.07 | 9 | CQLAKTCPV | p53.135 | 0.0240 |
| 1325.01 | 9 | RLPEAAPPV | p53.65L2 | 0.0640 |
| 1325.02 | 9 | GLAPPQHLV | p53.187V9 | 0.0130 |
| 1325.04 | 9 | KMAELVHFL | MAGE3.112M2 | 0.2100 |
| 1325.05 | 9 | KLAELVHFL | MAGE3.112L2 | 0.2500 |
| 1326.01 | 9 | CLLAKTCPV | p53.135L2 | 0.0400 |
| 1326.02 | 9 | KLSQHMTEV | p53.164L2 | 0.0410 |
| 1326.04 | 9 | ELAPWAPV | p53.68L2V9 | 0.0860 |
| 1326.06 | 10 | QLAKTCPVQV | p53.136 | 0.0320 |
| 1326.08 | 9 | HLTEVVRRV | p53.168L2 | 0.0180 |
| 1339.01 | 11 | KTYQGSYGFRL | p53.101 | 0.0028 |
| 1329.03 | 10 | VVVPYEPPEV | p53.216 | 0.0081 |
| 13Z9.14 | 9 | BQLAKTBPV | p53.135B1B7 | 0.0490 |
| 1329.15 | 9 | BLLAKTBPV | p53.135B1L2B7 | 0.1100 |
| 1330.01 | 9 | QIIGYVIGT | CEA.78 | 0.0160 |
| 1330.02 | 9 | QLIGYVIGV | CEA.78L2V9 | 0.5300 |
| 1330.05 | 9 | YVCGIQNSV | CEA.569 | 0.0510 |
| 1330.06 | 9 | YLCGIQNSV | CEA.569L2 | 0.1000 |
| 1330.07 | 9 | ATVGIMIGV | CEA.687 | 0.1400 |
| 1330.08 | 9 | ALVGIMIGV | CEA.687L2 | 0.5000 |
| 1330.09 | 10 | VLYGPDDPTI | CEA.411 | 0.0170 |
| 1330.10 | 10 | VLYGPDDPTV | CEA.411V10 | 0.0310 |
| 1331.02 | 9 | DLMLSPDDV | p53.42V9 | |
| 1331.03 | 9 | ALMLSPDDI | p53.42A1 | |
| 1331.04 | 9 | ALMLSPDDV | p53.42A1V9 | |
| 1331.45 | 9 | DLMLSPADI | p53.42A7 | |
| 1331.06 | 9 | DLMLSPADV | p53.42A7V9 | |
| 1331.07 | 9 | DLMLSPDAI | p53.42A8 | |
| 1331.08 | 9 | DLMLSPDAV | p53.42A8V9 | |

To identify peptides , class I antigen isolation, and isolation and sequencing of naturally processed peptides was carried out as described in the related applications. These peptides were then used to define specific binding motifs for each of the following alleles A3.2, A1, A11, and A24.1. These motifs are described above, The motifs described in Tables 6-9, below, are defined from pool sequencing data of naturally processed peptides as described in the related applications.

**TABLE 6**

| HLA-A3.2 Allele-Specific Motif | |
|---|---|
| Conserved | |
| Position | Residues |
| | |
| 1 | - |
| 2 | V,L,M |
| 3 | Y,D |
| 4 | - |
| 5 | - |
| 6 | - |
| 7 | I |
| 8 | Q,N |
| 9 | K |
| 10 | K |

**TABLE 7**

| HLA-A1 Allele-Specific Motif | |
|---|---|
| Conserved | |
| Position | Residues |
| 1 | - |
| 2 | S, T |
| 3 | D,E |
| 4 | P |
| 5 | - |
| 6 | - |
| 7 | L |
| 8 | - |
| 9 | Y |

**TABLE 8**

| HLA-A11 Allele-Specific Motif | |
|---|---|
| Conserved | |
| Position | Residues |
| 1 | - |
| 2 | T,V |
| 3 | M,F |
| 4 | - |
| 5 | - |
| 6 | - |
| 7 | - |
| 8 | Q |
| 9 | K |
| 10 | K |

**Table 9**

| HLA-A24.1 Allele-Specific Motif | |
|---|---|
| Conserved | |
| Position | Residues |
| 1 | - |
| 2 | Y |
| 3 | I,M |
| 4 | D,E,G,K,P |
| 5 | L,M,N |
| 6 | V |
| 7 | N,V |
| 8 | A,E,K,Q,S |
| 9 | F,L |
| 10 | F,A |

### Example 2

Using the motifs identified above for various MHC class I allele amino acid sequences from various pathogens and tumor-related proteins were analyzed for the presence of these motifs. Screening was carried out described in the related applications. Table 10 provides the results of searches of the antigens.

**TABLE 10**

| Peptide. | AA | Sequence | Source | A*0301 | A*1101 |
|---|---|---|---|---|---|
| 28.0719 | 10 | ILEQWVAGRK | HDV.nuc.16 | 0.0170 | 0.0012 |
| 28.0727 | 10 | LSAGGKNLSK | HDV.nuc. 115 | 0.0097 | 0.0150 |
| 1259.02 | 11 | STDTVDTVLEK | FIu.HA.29 | 0.0001 | 0.0676 |
| 1259.04 | 9 | GIAPLQLGK | Flu.HA.63 | 0.6100 | 0.2000 |
| 1259.06 | 10 | VTAACSHAGK | Flu.HA. 149 | 0.0380 | 0.0490 |
| 1259.08 | 9 | GIHHPSNSK | FIu.HA.195 | 0.1300 | 0.0140 |
| 1259.10 | 10 | RMNYYWTLLK | Flu.HA.243 | 2.5000 | 2.3000 |
| 1259.12 | 11 | ITNKVNSVIEK | Flu.HA.392 | 0.0200 | 0.0670 |
| 1259.13 | 11 | KMNIQFTAVGK | Flu.HA.402 | 0.0280 | 0.0092 |
| 1259.14 | 9 | NIQFTAVGK | Flu.HA.404 | 0.0017 | 0.0330 |
| 1259.16 | 11 | AVGKEFNKLEK | Flu.HA.409 | 0.0210 | 0.0460 |
| 1259.19 | 11 | KVKSQLKNNAK | Flu.HA.465 | 0.0470 | 0.0031 |
| 1259.20 | 11 | SVRNGTYDYPK | Flu.HA.495 | 0.0410 | 0.1400 |
| 1259.21 | 9 | SIIPSGPLK | Flu.VMT1.13 | 0.7800 | 8.8000 |
| 1259.25 | 10 | RMVLASTTAK | Flu.VMT1.178 | 0.5500 | 0.0350 |
| 1259.26 | 9 | MVLASTTAK | Flu.VMT1.179 | 1.7000 | 1.4000 |
| 1259.28 | 10 | RMGVQMQRFK | Flu.VMT1.243 | 0.1000 | 0.0059 |
| 1259.33 | 10 | ATEIRASVGK | Flu.VNUC.22 | 0.1400 | 0.3000 |
| 1259.37 | 11 | TMVMELVRMIK | Flu.VNUC.188 | 0.0890 | 0.0310 |
| 1259.43 | 10 | RVLSFIKGTK | Flu.VNUC.342 | 0.8000 | 0.0830 |
| F119.01 | 9 | MSLQRQFLR | ORF3P | 0.2000 | 0.7200 |
| F119.02 | 9 | LLGPGRPYR | TRP.197 | 0.0190 | 0.0091 |
| F119.03 | 9 | LLGPGRPYK | TRP.197K9 | 2.2000 | 0.6800 |

## Claims

1. An isolated immunogenic peptide consisting of p53 sequence SMPPPGTRV (SEQ ID NO: 4).

2. A composition comprising the peptide of claim 1.

3. The composition of claim 2, comprising a further immunogenic peptide having an HLA allele-specific binding motif, which immunogenic peptide is selected from the group consisting of the peptides listed in table 5 and table 10.

4. The composition of claim 2, wherein the isolated immunogenic peptide is linked to a T helper peptide.

5. The composition of claim 2, further comprising a PADRE molecule.

6. The composition of any one of claims 2-5, further comprising a lipid.

7. The composition of any one of claims 2-5, further comprising a liposome.

8. The composition of claim 2, wherein said peptide is linked to a spacer molecule.

9. The composition of any one of claims 2-5, further comprising a carrier.

10. The composition of any one of claims 2-5, further comprising an antigen presenting cell.

11. An isolated nucleic acid encoding the peptide of claim 1.

12. The isolated nucleic acid of claim 11, wherein said nucleic acid further encodes an immunogenic peptide having an HLA allele-specific binding motif, which immunogenic peptide is selected from the group consisting of the peptides listed in table 5 and table 10.

13. The isolated nucleic acid of claim 11, wherein said nucleic acid further encodes a T helper peptide.

14. The isolated nucleic acid of claim 11, wherein said nucleic acid further encodes a PADRE molecule.

15. The isolated nucleic acid of claim 12 which comprises a minigene construct encoding the peptide of claim 1 and further encoding an immunogenic peptide having an HLA allele-specific binding motif, which immunogenic peptide is selected from the group consisting of the peptides listed in table 5 and table 10.

16. The isolated nucleic acid of claim 15, wherein said minigene construct comprises a helper T lymphocyte epitope, a signal sequence and/or an endoplasmic reticulum retention signal.

17. A bicistronic expression vector comprising the minigene of claim 15 and a sequence encoding at least one protein or polypeptide included to enhance or decrease immunogenicity.

18. A composition comprising the nucleic acid of any one of claims 11-16 or the vector of claim 17.

19. The composition of claim 18, further comprising a cationic lipid.

20. The composition of claim 18, further comprising a carriers.

21. The use of the peptide of claim 1 in the manufacture of a medicament for the prevention or treatment of cancer.

22. The use according to 21, wherein CTL cells are generated ex vivo using said peptide in combination with a patient's CTL precursor cells.

23. The use of the nucleic acid of any of claims 11-16 or the vector of claim 18 in the manufacture of a medicament for the prevention or treatment of cancer.

24. A peptide according to claim 1 for use in the prevention or treatment of cancer.

25. A nucleic acid according to any one of claims 11 to 16 or a vector according to claim 17 for use in the prevention or treatment of cancer.

## Patentansprüche

1. Isoliertes immunogenes Peptid, das aus der p53-Sequenz SMPPPGTRV (Seq.-ID Nr. 4) besteht.

2. Zusammensetzung, umfassend das Peptid nach Anspruch 1.

3. Zusammensetzung nach Anspruch 2, umfassend ein weiteres immunogenes Peptid mit einem HLA-Allel-spezifischen Bindungsmotiv, wobei das immunogene Peptid aus der aus den in Tabelle 5 und Tabelle 10 aufgelisteten Peptiden bestehenden Gruppe ausgewählt ist.

4. Zusammensetzung nach Anspruch 2, worin das isolierte immunogene Peptid an ein T-Helfer-Peptid gebunden ist.

5. Zusammensetzung nach Anspruch 2, das weiters ein PADRE-Molekül umfasst.

6. Zusammensetzung nach einem der Ansprüche 2 bis 5, das weiters ein Lipid umfasst.

7. Zusammensetzung nach einem der Ansprüche 2 bis 5, das weiters ein Liposom umfasst.

8. Zusammensetzung nach Anspruch 2, worin das Peptid an ein Spacer-Molekül gebunden ist.

9. Zusammensetzung nach einem der Ansprüche 2 bis 5, das weiters einen Träger umfasst.

10. Zusammensetzung nach einem der Ansprüche 2 bis 5, das weiters eine antigenpräsentierende Zelle umfasst.

11. Isolierte Nucleinsäure, die für das Peptid nach Anspruch 1 kodiert.

12. Isolierte Nucleinsäure nach Anspruch 11, worin die Nucleinsäure weiters für ein immunogenes Peptid mit einem HLA-Allel-spezifischen Bindungsmotiv kodiert, wobei das immunogene Peptid aus der aus den in Tabelle 5 und Tabelle 10 aufgelisteten Peptiden bestehenden Gruppe ausgewählt ist.

13. Isolierte Nucleinsäure nach Anspruch 11, worin die Nucleinsäure weiters für ein T-Helfer-Peptid kodiert.

14. Isolierte Nucleinsäure nach Anspruch 11, worin die Nucleinsäure weiters für ein PADRE-Molekül kodiert.

15. Isolierte Nucleinsäure nach Anspruch 12, umfassend ein Minigen-Konstrukt, das für das Peptid nach Anspruch 1 kodiert und weiters für ein immunogenes Peptid mit einem HLA-Allel-spezifischen Bindungsmotiv kodiert, wobei das immunogene Peptid aus der aus den in Tabelle 5 und Tabelle 10 aufgelisteten Peptiden bestehenden Gruppe ausgewählt ist.

16. Isolierte Nucleinsäure nach Anspruch 15, worin das Minigen-Konstrukt ein Helfer-T-Lymphozyten-Epitop, eine Signalsequenz und/oder ein Retentionssignal des endoplasmatischen Retikulums umfasst.

17. Bicistronischer Expressionsvektor, umfassend ein Minigen nach Anspruch 15 und eine Sequenz, die für zumindest ein Protein oder Polypeptid kodiert, das inkludiert ist, um die Immunogenität zu verstärken oder zu verringern.

18. Zusammensetzung, umfassend eine Nucleinsäure nach einem der Ansprüche 11 bis 16 oder einen Vektor nach Anspruch 17.

19. Zusammensetzung nach Anspruch 18, die weiters ein kationisches Lipid umfasst.

20. Zusammensetzung nach Anspruch 18, die weiters einen Träger umfasst.

21. Verwendung des Peptids nach Anspruch 1 zur Herstellung eines Medikaments zur Prävention oder Behandlung von Krebs.

22. Verwendung nach Anspruch 21, worin CTL-Zellen ex vivo unter Verwendung des Peptids in Kombination mit CTL-Vorläufer-Zellen eines Patienten erzeugt werden.

23. Verwendung einer Nucleinsäure nach einem der Ansprüche 11 bis 16 oder eines Vektors nach Anspruch 18 zur Herstellung eines Medikaments zur Prävention oder Behandlung von Krebs.

24. Peptid nach Anspruch 1 zur Verwendung bei der Prävention oder der Behandlung von Krebs.

25. Nucleinsäure nach einem der Ansprüche 11 bis 16 oder Vektor nach Anspruch 17 zur Verwendung bei der Prävention oder der Behandlung von Krebs.

## Revendications

1. Peptide immunogène isolé consistant en p53 séquence SMPPPGTRV (SEQ ID NO: 4).

2. Composition comprenant le peptide de la revendication 1.

3. Composition selon la revendication 2, comprenant un autre peptide immunogène ayant un motif de liaison HLA allèle-spécifique, ledit peptide immunogène étant sélectionné dans le groupe comprenant les peptides énumérés dans le tableau 5 et le tableau 10.

4. Composition selon la revendication 2, dans laquelle le peptide immunogène isolé est lié à un peptide T auxiliaire.

5. Composition selon la revendication 2, comprenant en outre une molécule PADRE.

6. Composition selon l'une quelconque des revendications 2 à 5, comprenant en outre un lipide.

7. Composition selon l'une quelconque des revendications 2 à 5, comprenant en outre un liposome.

8. Composition selon la revendication 2, dans laquelle ledit peptide est lié à une molécule d'espacement.

9. Composition selon l'une quelconque des revendications 2 à 5, comprenant en outre un support.

10. Composition selon l'une quelconque des revendications 2 à 5, comprenant en outre une cellule de présentation d'antigène.

11. Acide nucléique isolé codant pour un peptide de la revendication 1.

12. Acide nucléique isolé selon la revendication 11, dans lequel ledit acide nucléique code en outre pour un peptide immunogène ayant un motif de liaison HLA allèle-spécifique, ledit peptide immunogène est sélectionné dans le groupe comprenant les peptides énumérés dans le tableau 5 et le tableau 10.

13. Acide nucléique isolé selon la revendication 11, dans lequel ledit acide nucléique code en outre pour un peptide T auxiliaire.

14. Acide nucléique isolé selon la revendication 11, dans lequel ledit acide nucléique code en outre pour une molécule PADRE.

15. Acide nucléique isolé selon la revendication 12, qui comprend un produit de synthèse minigène codant pour le peptide de la revendication 1, et codant en outre pour un peptide immunogène ayant un motif de liaison HLA allèle-spécifique, ledit peptide immunogène est sélectionné dans le groupe comprenant les peptides énumérés dans le tableau 5 et le tableau 10.

16. Acide nucléique isolé selon la revendication 15, dans lequel ledit produit de synthèse minigène comprend un épitope de lymphocyte T auxiliaire, une séquence de signaux et/ou un signal de retenue de réticulum endoplasmique.

17. Vecteur d'expression bicistronique comprenant le minigène de la revendication 15 et une séquence codant pour au moins une protéine ou polypeptide incluse pour augmenter ou diminuer l'immunogénicité.

18. Composition comprenant l'acide nucléique selon l'une quelconque des revendications 11 à 16 ou le vecteur de la revendication 17.

19. Composition selon la revendication 18, comprenant en outre un lipide cationique.

20. Composition selon la revendication 18, comprenant en outre un support.

21. Utilisation du peptide selon la revendication 1 dans la fabrication d'un médicament pour la prévention ou le traitement du cancer.

22. Utilisation selon la revendication 21, dans laquelle des cellules CTL sont produites ex vivo en utilisant ledit peptide en combinaison avec des cellules précurseurs du CTL d'un patient.

23. Utilisation de l'acide nucléique selon l'une quelconque des revendications 11 à 16 ou le vecteur de la revendication 18 dans la fabrication d'un médicament pour la prévention ou le traitement du cancer.

24. Peptide selon la revendication 1, pour utilisation dans la prévention ou le traitement du cancer.

25. Acide nucléique selon l'une quelconque des revendications 11 à 16 ou un vecteur selon la revendication 17 pour utilisation dans la prévention ou le traitement du cancer.
